# EUROPEAN PATENT APPLICATION

(11) **EP 3 097 846 A1**
(43) Date of publication of application: **30.11.2016**
(21) Application number: 15741004.4
(22) Date of filing: 19.01.2015
(51) Int. Cl.: A61B 1/06, G02B 23/26

(54) **LIGHT SOURCE SYSTEM FOR ENDOSCOPE**

(30) Priority: 23.01.2014 JP 2014010726
(71) Applicant: Olympus Corporation, Shibuya-ku Tokyo 151-0072 (JP)
(72) Inventor: TABATA, Motoki, Hachioji-shi Tokyo 192-8507 (JP); DAIDOJI, Bakusui, Hachioji-shi Tokyo 192-8507 (JP); OHARA, Satoshi, Hachioji-shi Tokyo 192-8507 (JP); SHINJI, Sho, Hachioji-shi Tokyo 192-8507 (JP); HANANO, Kazunari, Hachioji-shi Tokyo 192-8507 (JP)
(74) Representative: Winter, Brandl, Fürniss, Hübner, Röss, Kaiser, Polte - Partnerschaft mbB
(86) International application number: PCT/JP2015/051212
(87) International publication number: WO 2015/111540

(57) **Abstract**

A first light source module 20 and an irradiation module 50, 70 are mutually combined. By the combination, illumination light corresponding to a purpose of use is emitted. A relative distance between a light source-side emission unit 23 and an irradiation-side incidence portion 51, 71 in an optical axis direction is adjusted as desired in accordance with the irradiation module 50, 70, which is connected to the first light source module 20.

## Description

### Technical Field

The present invention relates to an endoscope light source system in which a light source module and an irradiation module are mutually combined, and thereby light corresponding, to a purpose of use is emitted.

### Background Art

For example, an observation device, such as an endoscope, includes a light source system. Conventionally, in the structure of the light source system, light is emitted from a lamp light source such as a xenon lamp, and is guided by a bundle fiber which is formed by bundling optical fibers. On the other hand, in the structure of light source systems in recent years, a semiconductor light source such as an LD, and a single optical fiber are utilized. In this structure, light is emitted from the light source such as the LD, and is guided by the single optical fiber. Then, the color, light intensity distribution, etc. of the light are converted by an optical conversion member disposed at a distal end portion of a light guide member, and the light in the converted state is emitted. The LD is suited to special optical observation utilizing light in a narrow band, and reduction in size and enhancement in efficiency of the light source system can be realized by the LD.

In such the light source system, a light source module and an irradiation module are mutually combined, and thereby light corresponding to a purpose of use is emitted. In general, a diameter of a core of an optical fiber is very small. Thus, when the irradiation module is connected to the light source module in the state in which a single optical fiber is used, it is required that the optical fiber on the irradiation module side is precisely connected to the optical fiber on the light module side.

A technique relating to such connection is disclosed, for example, in Patent Literature 1. In Patent Literature 1, graded-index (GI) collimators are disposed at an end portion of the optical fiber on the light source module side and at an end portion of the optical fiber on the irradiation module side. Emission light, which is emitted from the end portion of the optical fiber on the light source module side, is diverged by the GI collimator. This diverged emission light is made incident on the GI collimator on the irradiation module side and is focused by the GI collimator, and the light in the focused state enters the optical fiber. Thereby, the effect of optical axis misalignment is reduced, and the optical fibers are precisely connected.

### Citation List

### Patent Literature

Patent Literature 1: Jpn. Pat. Appln. KOKAI Publication No. 2011-152370

### Summary of Invention

### Technical Problem

The content disclosed in Patent Literature 1 is adaptive to only a single fiber scope functioning as an irradiation module using a single optical fiber, and no consideration is given to adaptation to a bundle fiber scope functioning as an irradiation module using a bundle fiber. Thus, even if the bundle fiber scope is combined with the light source module, the bundle fiber scope cannot properly optically connected to the light source module. In this manner, the light source module cannot secure compatibility with the single fiber scope and the bundle fiber scope.

In other words, there is concern that irradiation modules having mutually different optical functions cannot properly optically be connected to the light source module, and each irradiation module cannot exhibit performance.

The present invention has been made in consideration of the above circumstance, and the object of the invention is to provide an endoscope light source system which can exhibit performance even if respective irradiation modules have mutually different optical functions.

### Solution to Problem

An aspect of an endoscope light source system of the invention is an endoscope light source system in which a light source module and an irradiation module, which is mechanically detachably attached to the light source module, are combined and thereby illumination light corresponding to a purpose of use is emitted, the light source module includes a light source unit configured to emit light-source light; a light source-side emission unit configured to convert an optical characteristic of the light-source light, and to emit the light with the converted optical characteristic as primary emission light; and a light source-side connection hole portion disposed on an optical axis of the light source-side emission unit, and made common to various kinds of the irradiation modules which have mutually different optical functions, the irradiation module includes an irradiation-side incidence portion on which the primary emission light emitted from the light source-side emission unit is made incident; an irradiation-side connection portion configured to be connected to the light source-side connection hole portion, such that the irradiation-side incidence portion is disposed coaxial with the light source-side emission unit, and the primary emission light emitted from the light source-side emission unit is made incident on the irradiation-side incidence portion; a light guide member configured to guide the primary emission light made incident on the irradiation-side incidence portion; and an irradiation-side emission unit configured to convert an optical characteristic of the primary emission light guided by the light guide member, and to emit the light with the converted optical characteristic as the illumination light to an outside, wherein relative distance between the light source-side emission unit and the irradiation-side incidence portion in an optical axis direction is adjusted, as desired, in accordance with the irradiation module which is connected to the light source module.

### Brief Description of Drawings

FIG. 1A is a schematic view of an endoscope light source system according to a first embodiment of the present invention.
FIG. 1B is a view illustrating a state in which a first light source module shown in FIG. 1A is connected to a first irradiation module.
FIG. 1C is a view illustrating a state in which the first light source module shown in FIG. 1A is connected to a second irradiation module.
FIG. 2A is a schematic view of an endoscope light source system according to a first modification of the first embodiment.
FIG. 2B is a view illustrating a state in which a first light source module shown in FIG. 2A is connected to a first irradiation module.
FIG. 2C is a view illustrating a state in which the first light source module shown in FIG. 2A is connected to a second irradiation module.
FIG. 3A is a schematic view of an endoscope light source system according to a second modification of the first embodiment.
FIG. 3B is a cross-sectional view taken along line 3B-3B shown in FIG. 3A.
FIG. 3C is a view illustrating a modification of a cylinder portion shown in FIG. 3A.
FIG. 3D is a cross-sectional view taken along line 3D-3D shown in FIG. 3C.
FIG. 4A is a schematic view of an endoscope light source system according to a second embodiment of the present invention.
FIG. 4B is a view illustrating a state in which a first light source module shown in FIG. 4A is connected to a first irradiation module.
FIG. 4C is a view illustrating a state in which the first light source module shown in FIG. 4A is connected to a second irradiation module.

### Description of Embodiments

Hereinafter, embodiments of the present invention will be described in detail with reference to the accompanying drawings. Incidentally, in some drawings, depiction of some of members is omitted for the purpose of clearer illustration.

### [First Embodiment]

### [Structure]

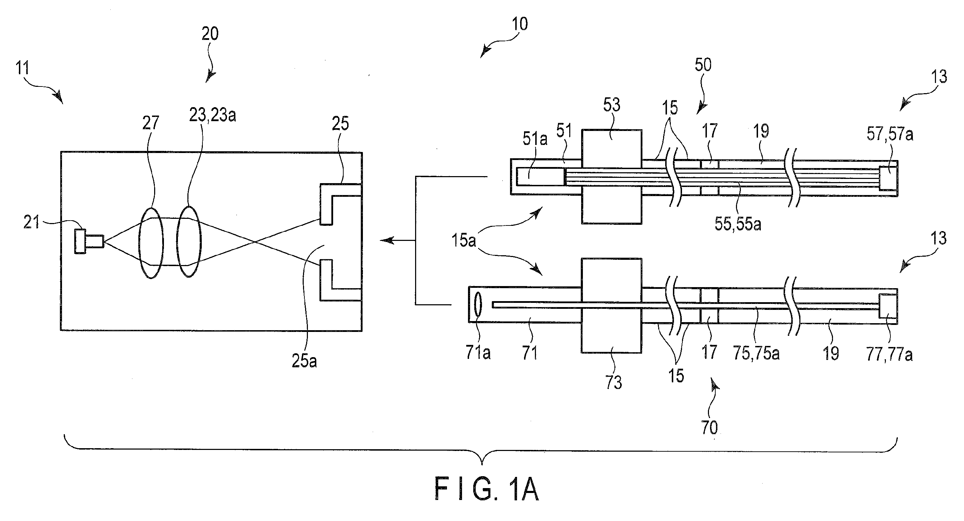

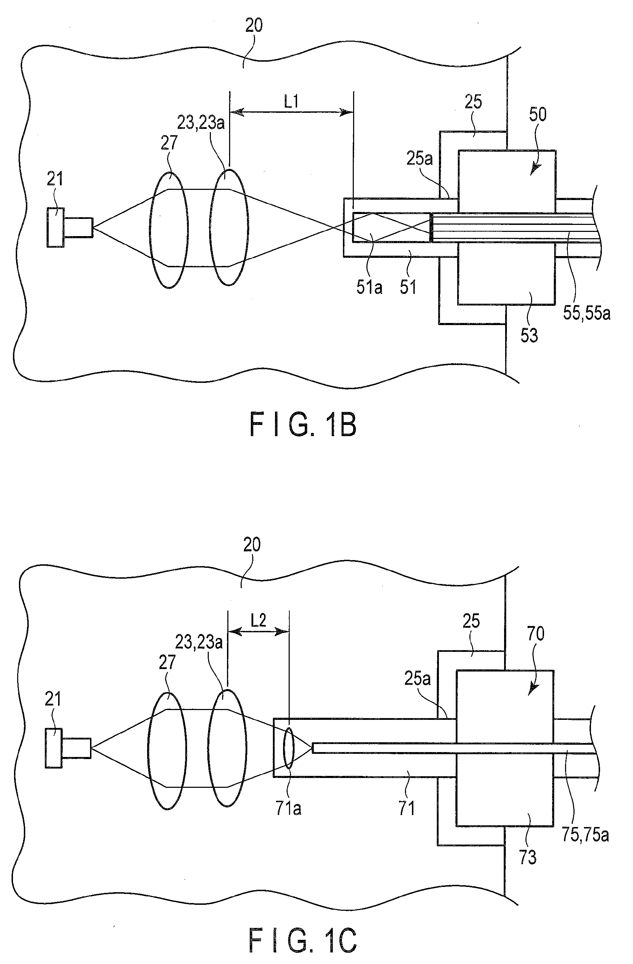

A first embodiment will be described with reference to FIG. 1A, FIG. 1B and FIG. 1C.

### [Structure of Endoscope Light Source System 10]

An endoscope light source system 10 as illustrated in FIG. 1A includes a light source module, and an irradiation module which can be mechanically detachably attached to the light source module. As illustrated in FIG. 1A, the endoscope light source system 10 is composed of, for example, one light source module (first light source module 20) and two irradiation modules (first irradiation module 50 and second irradiation module 70). The respective irradiation modules 50 and 70 are various kinds of modules having, for example, mutually different optical functions. In addition, as illustrated in FIG. 1A, FIG. 1B and FIG. 1C, the first light module 20 and the irradiation module 50, 70 are mutually combined such that when the first irradiation module 50 is attached to the first light source module 20, the second irradiation module 70 is detached from the first light source module 20, and such that when the second irradiation module 70 is attached to the first light source module 20, the first irradiation module 50 is detached from the first light source module 20. By this combination, illumination light corresponding to a purpose of use is emitted from the irradiation module 50, 70, which is connected to the first light source module 20. Furthermore, the first light source module 20 is a common member which is shared and made common between the first irradiation module 50 and second irradiation module 70.

The first light source module 20 is mounted on, for example, a light source device 11, and the irradiation module 50, 70 is mounted on, for example, an endoscope 13 which is detachably attached to the light source device 11.

### [Light Source Module]

Hereinafter, referring to FIG. 1A, FIG. 1B and FIG. 1C, a description is given of a concrete structure of the light source device by taking the first light source module 20 as an example.

As illustrated in FIG. 1A, FIG. 1B and FIG. 1C, the first light source module 20 includes a light source unit 21 which emits light-source light, and a light source-side emission unit 23 which converts an optical characteristic of the light-source light, and emits the light with the converted optical characteristics as primary emission light. The first light source module 20 further includes a light source-side connection hole portion 25 which is disposed on an optical axis of the light source-side emission unit 23, and is made common to various kinds of irradiation modules 50, 70, which have, for example, mutually different optical functions.

The light source unit 21 as illustrated in FIG. 1A, FIG. 1B and FIG. 1C includes, for example, an LD which emits a laser beam that is light-source light. The light source unit 21 emits, for example, white light, or special light which can improve the visibility of a specific object of observation. Thus, although illustration is omitted, the light source unit 21 includes, for example, an LD which emits a laser beam with a wavelength of 405 nm, an LD which emits a laser beam with a wavelength of 445 nm, an LD which emits a laser beam with a wavelength of 515 nm, and an LD which emits a laser beam with a wavelength of 650 nm. Although illustration is omitted, the light source unit 21 further includes a light coupler which combines the laser beams emitted from the respective LDs, and an emission unit which emits light combined by the light coupler. For example, the laser beams of 445 nm, 515 nm and 650 nm are combined to produce white light. For example, the laser beams of 405 nm and 515 nm are combined to produce NBI special light which enables observation with good contrast of, for example, a blood vessel, and enables easy discovery of, for example, cancer. These lights are emitted as light-source lights.

As illustrated in FIG. 1A, FIG. 1B and FIG. 1C, the first light source module 20 further includes a collimation member 27 which converts light-source light, which is emitted from the light source unit 21, to a parallel beam. This collimation member 27 includes, for example, a first lens. The collimation member 27 is disposed in front of the light source unit 21 and in rear of the light source-side emission unit 23 in the direction of travel of light.

As illustrated in FIG. 1A, FIG. 1B and FIG. 1C, the light source-side emission unit 23 includes a light focusing member 23a which focuses the primary emission light to a desired part of the irradiation module 50, 70, when the light source-side connection hole portion 25 is connected to an irradiation-side connection portion 53, 73 (to be described later) of the irradiation module 50, 70. This light focusing member 23a includes, for example, a second lens. The light focusing member 23a is disposed in front of the collimation member 27 in the direction of travel of light.

A relationship, 1.5 < focal distance f2 of the second lens/focal distance f1 of the first lens < 2.5, is established with respect to the first lens of the collimation member 27 and the second lens of the light focusing member 23a. By this relationship, when an emission portion (not shown) of the light source unit 21 and a second light guide member 75, which the second irradiation module 70 includes, are optical fibers having an identical core diameter and an identical NA, the convergence of emission light emitted from the emission portion is maintained and the light in the irradiation module becomes focused light with a less angle than the NA of the second light guide member 75.

As illustrated in FIG. 1A, FIG. 1B and FIG. 1C, the light source-side connection hole portion 25 functions as a receptacle portion of the light source device 11, a connecting connector 15a, which is disposed on a universal cord 15 of the endoscope 13, is attached/detached to/from the light source-side connection hole portion 25. The light source-side connection hole portion 25 is shared and made common between a first irradiation-side connection portion 53 and a second irradiation-side connection portion 73, such that the light source-side connection hole portion 25 may be detachably connected to the first irradiation-side connection portion 53 mounted on the first irradiation module 50 and to the second irradiation-side connection portion 73 mounted on the second irradiation module 70. The light source-side connection hole portion 25 is a common member to the first irradiation-side connection portion 53 and second irradiation-side connection portion 73. Thus, the light source-side connection hole portion 25, which is connected to the first irradiation-side connection portion 53, is the same part as the light source-side connection hole portion 25 which is connected to the second irradiation-side connection portion 73, and is disposed at the same position as the light source-side connection hole portion 25 which is connected to the second irradiation-side connection portion 73.

As illustrated in FIG. 1A, FIG. 1B and FIG. 1C, the light source-side connection hole portion 25 is disposed coaxial with, for example, the light focusing member 23a, and is disposed on the same axis as the position at which the light focused by the light focusing member 23a is focused.

As illustrated in FIG. 1A, FIG. 1B and FIG. 1C, the light source-side connection hole portion 25 includes a through-hole portion 25a through which a first irradiation-side incidence portion 51 mounted on the first irradiation module 50 and a second irradiation-side incidence portion 71 mounted on the second irradiation module 7 0 are configured to penetrate. The through-hole portion 25a is disposed coaxial with, for example, the light focusing member 23a.

### [Irradiation Module]

As described above, the irradiation modules include the first irradiation module 50 and second irradiation module 70 as illustrated in FIG. 1A, FIG. 1B and FIG. 1C. A brief description will be given below of common parts between the first irradiation module 50 and second irradiation module 70.

The irradiation module 50, 70 includes the irradiation-side incidence portion 51, 71 on which the primary emission light emitted from the light source-side emission unit 23 is made incident, and the irradiation-side connection portion 53, 73 which is connected to the light source-side connection hole portion 25 such that the irradiation-side incidence portion 51, 71 is disposed coaxial with the light source-side emission unit 23, and the primary emission light emitted from the light source-side emission unit 23 is made incident on the irradiation-side incidence portion 51, 71. The irradiation-side incidence portion 51, 71 and irradiation-side connection portion 53, 73 are disposed, for example, within the connecting connector 15a disposed in the universal cord 15 of the endoscope 13.

The irradiation module 50, 70 further includes a light guide member 55, 75 which guides the primary emission light that is incident on the irradiation-side incidence portion 51, 71 and an irradiation-side emission unit 57, 77 which converts the optical characteristic of the primary emission light guided by the light guide member 55, 75, and emits secondary emission light, which is illumination light, to an outside. The light guide member 55, 75 is disposed in the inside of the universal cord 15, an operation section 17 and a soft insertion section 19 of the endoscope 13. The irradiation-side emission unit 57, 77 is disposed in the inside of a distal end portion of the insertion section 19.

A greatest difference between the first irradiation module 50 and second irradiation module 70 is that their optical functions, for example, are different from each other, and, specifically, the kinds of their light guide members 55 and 75 are different from each other. To be more specific, the size of an incidence end face of the light guide member 55, 75, on which the primary emission light is incident, is different between the irradiation modules.

Thus, for example, in the first irradiation module 50, the first light guide member 55 includes a bundle fiber 55a with a large size of the incidence end face. The bundle fiber 55a is formed by bundling a plurality of optical fiber strands. This first irradiation module 50 functions as a bundle fiber scope.

For example, in the second irradiation module 70, the second light guide member 75 includes a single optical fiber 75a with a small size of the incidence end face. This second irradiation module 70 functions as a single fiber scope.

The endoscope 13, in which the above-described first irradiation module 50 is mounted, is a separate body from the endoscope 13 in which the second irradiation module 70 is mounted.

Hereinafter, concrete structures of the first irradiation module 50 and second irradiation module 70 will be described.

[First Irradiation Module 50 (Bundle Fiber Scope)]

As illustrated in FIG. 1A and FIG. 1B, in the first irradiation module 50, the first irradiation-side incidence portion 51, the first irradiation-side connection portion 53, the first light guide member 55 and the first irradiation-side emission unit 57 are mounted.

As illustrated in FIG. 1A and FIG. 1B, the first irradiation-side incidence portion 51 includes a glass rod 51a on which the primary emission light focused by the light focusing member 23a is made incident, when the first irradiation-side connection portion 53 is connected to the light source-side connection hole portion 25. The glass rod 51a is optically connected to an incidence end face disposed at one end portion of the bundle fiber 55a. The glass rod 51a includes a core portion (not shown) disposed at a central part of the glass rod 51a, and a clad portion (not shown) disposed in a manner to cover the core portion. The refractive index of the clad portion is lower than the refractive index of the core portion. Thus, the primary emission light is reflected by an interface between the core portion and clad portion, confined in the core portion, and guided by the core portion. Thereby, the glass rod 55a confines the primary emission light in the inside of the glass rod 51a, and transmits the primary emission light to the bundle fiber 55a without leaking the primary emission light. The diameter of the glass rod 51a is substantially equal to the diameter of the bundle fiber 55a.

The glass rod 51 uniformizes the light intensity in the cross section in a direction perpendicular to the optical axis of the primary emission light. In general, the light intensity of a laser bean is strong at a central part of the laser beam, and becomes weaker away from the central part. In this manner, the light intensity of the laser beam is nonuniform. If the laser beam is directly made incident in the bundle fiber 55a in this state, a variance occurs among the amounts of light incident on the respective optical fibers of the bundle fiber 55a. The tendency of variance is propagated to the other end portion (first irradiation-side emission unit 57) of the bundle fiber 55a. Consequently, a deviation occurs in the light intensity of the laser beam emitted from the bundle fiber 55a, and nonuniformity in luminance or nonuniformity in light distribution occurs in illumination light. However, by the glass rod 51a, the laser beam that is the primary emission light is repeatedly reflected within the glass rod 51a, and thus the laser beam is incident, with no variance, on the entire incidence end face of the bundle fiber 55a. Hence, the deviation in light intensity of the laser beam is eliminated, and the light intensity becomes uniform. Therefore, nonuniformity in luminance or nonuniformity in light distribution is prevented.

As illustrated in FIG. 1A and FIG. 1B, the glass rod 51a is disposed on one end portion side of the first irradiation-side incidence portion 51, and one end portion of the first light guide member 55, which is optically connected to the glass rod 51a, is disposed on the other end portion side of the first irradiation-side incidence portion 51. The other end portion of the first irradiation-side incidence portion 51 is coupled to the first irradiation-side connection portion 53.

As illustrated in FIG. 1A and FIG. 1B, the first irradiation-side connection portion 53 is detachably engaged with the light source-side connection hole portion 25. The first irradiation-side connection portion 53 is mechanically connected to the light source-side connection hole portion 25 such that one end portion of the first irradiation-side incidence portion 51 penetrates the through-hole portion 25a, and the other end portion of the first irradiation-side incidence portion 51 is placed in the through-hole portion 25a.

As illustrated in FIG. 1A and FIG. 1B, the first light guide member 55 includes the above-described bundle fiber 55a. Each of the optical fibers of the bundle fiber 55a includes a core portion (not shown) disposed at a central part of the single optical fiber, and a clad portion (not shown) disposed in a manner to cover the core portion. The refractive index of the clad portion is lower than the refractive index of the core portion. Thus, the primary emission light is reflected by an interface between the core portion and clad portion, confined in the core portion, and guided by the core portion. Thereby, the optical fiber confines the primary emission light in the inside of the optical fiber, and transmits the primary emission light to the first irradiation-side emission unit 57 without leaking the primary emission light. The diameter of the optical fiber is, for example, 20 *µ*m to 70 *µ*m. The diameter of the bundle fiber 55a is, for example, several 1 mm to 4 mm.

As illustrated in FIG. 1A, the first irradiation-side emission unit 57 includes an optical conversion member 57a which is disposed at the other end portion of the first irradiation module 50 and is optically connected to the other end portion of the first light guide member 55. The optical conversion member 57a includes a lens system which converts the primary emission light, which is emitted from the other end portion of the first light guide member 55, to illumination light having a desired light distribution and divergence angle, and irradiates the illumination light. In general, since the divergence angle of the light emitted from the other end portion of the first light guide member 55 is small, the optical conversion member 57a increases this divergence angle.

### [Second Radiation Module 70 (Single Fiber Scope)]

As illustrated in FIG. 1A and FIG. 1C, in the second irradiation module 70, the second irradiation-side incidence portion 71, the second irradiation-side connection portion 73, the second light guide member 75 and a second irradiation-side emission unit 77 are mounted.

As illustrated in FIG. 1A and FIG. 1C, the second irradiation-side incidence portion 71 includes a light focusing member 71a which further focuses the primary emission light, which was focused by the light focusing member 23a, on the single optical fiber 75, such that the primary emission light, which was focused by the light focusing member 23a, may be made incident on the single optical fiber 75 when the second irradiation-side connection portion 73 is connected to the light source-side connection hole portion 25. The light focusing member 71a is optically connected to one end portion of the single optical fiber 75a. In the single optical fiber 75a, the diameter of the core portion (not shown) is, for example, 50 *µ*m to 300 *µ*m. Thus, the light focusing member 71a prevents optical loss occurring due to positional displacement. The positional displacement includes displacement of the optical axis on the first light source module 20 side relative to the optical axis on the first irradiation module 50 side, and displacement of the first irradiation module 50 relative to the first light source module 20 in the optical axis direction. The optical loss indicates, for example, that the amount of primary emission light, which is incident on the fine single optical fiber 75a, decreases due to the positional displacement.

As illustrated in FIG. 1A and FIG. 1C, the light focusing member 71a and one end portion of the second light guide member 75, which is optically connected to the light focusing member 71a, are disposed on one end side of the second irradiation-side incidence portion 71. The second light guide member 75 is disposed on the other end portion side of the second irradiation-side incidence portion 71. The other end portion of the second irradiation-side incidence portion 71 is coupled to the second irradiation-side connection portion 73.

As illustrated in FIG. 1A, FIG. 1B and FIG. 1C, the second irradiation-side incidence portion 71 has, for example, the same thickness and same outer shape as the first irradiation-side connection portion 53. The second irradiation-side incidence portion 71 is longer than the first irradiation-side connection portion 53.

As illustrated in FIG. 1A and FIG. 1C, the second irradiation-side connection portion 73 is detachably engaged with the light source-side connection hole portion 25. The second irradiation-side connection portion 73 is mechanically connected to the light source-side connection hole portion 25 such that one end portion of the second irradiation-side incidence portion 71 penetrates the through-hole portion 25a, and the other end portion of the first irradiation-side incidence portion 51 is placed in the through-hole portion 25a.

As illustrated in FIG. 1A, FIG. 1B and FIG. 1C, the second irradiation-side connection portion 73 has the same thickness and same outer shape as the first irradiation-side connection portion 53.

As illustrated in FIG. 1A and FIG. 1C, the second light guide member 75 includes the above-described single optical fiber 75a. The single optical fiber 75a includes a core portion (not shown) disposed at a central part of the single optical fiber 75a, and a clad portion (not shown) disposed in a manner to cover the core portion. The diameter of the core portion is, for example, 50 *µ*m to 300 *µ*m. The refractive index of the clad portion is lower than the refractive index of the core portion. Thus, the primary emission light is reflected by an interface between the core portion and clad portion, confined in the core portion, and guided by the core portion. Thereby, the optical fiber confines the primary emission light in the inside of the optical fiber, and transmits the primary emission light to the second irradiation-side emission unit 77 without leaking the primary emission light.

As illustrated in FIG. 1A and FIG. 1C, the second irradiation-side emission unit 77 includes an optical conversion member 77a which is disposed at the other end portion of the second irradiation module 70 and is optically connected to the other end portion of the single optical fiber 75a. The optical conversion unit 77a converts, as desired, the wavelength and light distribution characteristics of the primary emission light, which is emitted from the other end portion of the single optical fiber 75a, and diffuses and emits the light as illumination light.

### [Relative Distance between Light Source-Side Emission Unit 23 and Radiation-Side Incidence Portion 51, 71 in Optical Axis Direction]

The relative distance between the light source-side emission unit 23 and the irradiation-side incidence portion 51, 71 in the optical axis direction is adjusted as desired in accordance with the irradiation module 50, 70, which is connected to the first light source-side module 20. Hereinafter, referring to FIG. 1B and FIG. 1C, a description is given of a relative distance L1 which is adjusted for the first irradiation module 50, and a relative distance L2 which is adjusted for the second irradiation module 70.

As illustrated in FIG. 1B, in the present embodiment, a relative distance L1 is specified (adjusted) such that, in the first irradiation module 50, the optical axis of the light focusing member 23a agrees with the optical axis of the glass rod 51a, and the glass rod 51a is located in front of the focal point of the light focusing member 23a, when the first irradiation-side connection portion 53 is connected to the light source-side connection hole portion 25 and the first irradiation module 50 is connected to the first light source module 20. Specifically, this relative distance L1 indicates a distance in the optical axis direction between the light focusing member 23a of the light source-side emission unit 23 and the incidence end face of the glass rod 51a of the first irradiation-side connection portion 53, on which the primary emission light focused by the light focusing member 23a is made incident. The incidence end face of the glass rod 51a functions as an incidence portion at which the primary emission light is made incident on the first irradiation module 50. When the first irradiation module 50 is connected to the first light source module 20, the position of the light focusing member 23a is fixed in advance, and thus the position of the incidence end face needs to be specified in accordance with the connection, such that the relative distance L1 is specified. Thus, in the present embodiment, the length of the first irradiation-side incidence portion 51 is specified such that the position is specified, or in other words, the relative distance L1 is specified. Specifically, the position of the incidence end face of the glass rod 51a relative to the light focusing member 23a is specified by the length of the first irradiation-side incidence portion 51 and the position of connection between the first irradiation-side connection portion 53 and the light source-side connection hole portion 25.

As illustrated in FIG. 1C, in the present embodiment, a relative distance L2 is specified (adjusted) such that, in the second irradiation module 70, the optical axis of the light focusing member 23a agrees with the optical axis of the light focusing member 71a, and the light focusing member 71a is located in rear of the focal point of the light focusing member 23a, when the second irradiation-side connection portion 73 is connected to the light source-side connection hole portion 25 and the second irradiation module 70 is connected to the first light source module 20. Specifically, this relative distance L2 indicates a distance in the optical axis direction between the light focusing member 23a of the light source-side emission unit 23 and the light focusing member 71a of the second irradiation-side connection portion 73, on which the primary emission light focused by the light focusing member 23a is made incident. The light focusing member 71a functions as an incidence portion at which the primary emission light is made incident on the second irradiation module 70. When the second irradiation module 70 is connected to the first light source module 20, the position of the light focusing member 23a is fixed in advance, and thus the position of the light focusing member 71a needs to be specified in accordance with the connection, such that the relative distance L2 is specified. Thus, in the present embodiment, the length of the second irradiation-side incidence portion 71 is specified such that the position is specified, or in other words, the relative distance L2 is specified. The relative distance L2 is shorter than the relative distance L1. Specifically, the position of the light focusing member 71a relative to the light focusing member 23a is specified by the length of the second irradiation-side incidence portion 71 and the position of connection between the second irradiation-side connection portion 73 and the light source-side connection hole portion 25.

In this manner, in the present embodiment, as illustrated in FIG. 1A, FIG. 1B and FIG. 1C, when the irradiation-side connection portion 53 , 73 is mechanically connected to the light source-side connection hole portion 25, the relative distance L1, L2 is adjusted. In particular, the length in the optical axis direction of the irradiation-side incidence portion 51, 71 relative to the irradiation-side connection portion 53, 73 is different between the irradiation modules, and thereby the relative distance L1, L2 is adjusted.

Specifically, the first light source module 20 is a common member to the first irradiation module 50 and second irradiation module 70. No matter which of the first irradiation module 50 and second irradiation module 70 is connected to the first light source module 20, the position of the light focusing member 23a and the positon of the light source-side connection hole portion 25 in the first light source module 20 are fixed and invariable. The position of connection of the first irradiation-side connection portion 53 to the light source-side connection hole portion 25 is identical to the position of connection of the second irradiation-side connection portion 73 to the light source-side connection hole portion 25. When the first irradiation-side connection portion 53 is connected to the light source-side connection hole portion 25, the other end portion of the first irradiation-side incidence portion 51 is placed in the through-hole portion 25a, and also when the second irradiation-side connection portion 73 is connected to the light source-side connection hole portion 25, the other end portion of the second irradiation-side incidence portion 71 is placed in the through-hole portion 25a. Thus, in the present embodiment, in the case in which the first irradiation module 50 is connected to the first light source module 20 and in the case in which the second irradiation module 70 is connected to the first light source module 20, the position of the incidence end face of the glass rod 51a of the first irradiation module 50 relative to the light focusing member 23a is different from the position of the light focusing member 71a relative to the light focusing member 23a. Specifically, the second irradiation-side incidence portion 71 is longer than the first irradiation-side incidence portion 51, such that the glass rod 51a is located farther from the light focusing member 23a, and the light focusing member 71a is located closer to the light focusing member 23a. Thus, in the present embodiment, the difference between the relative distance L1 and relative distance L2 is the difference between the length of the first irradiation-side incidence portion 51 and the length of the second irradiation-side incidence portion 71. In addition, the length of the first irradiation-side incidence portion 51 and the length of the second irradiation-side incidence portion 71 function as an adjustment mechanism which adjusts the relative distance L1, L2, as desired, in accordance with the irradiation module 50, 70, which is connected to the first light source module 20.

In this manner, the relative distance L1 is adjusted such that the first irradiation-side incidence portion 51 is located farther from the focal position of the primary emission light focused by the light focusing member 23a, when the first irradiation module 50 (bundle fiber scope) is connected to the first light source module 20. The relative distance L2 is adjusted such that the second irradiation-side incidence portion 71 is located closer to the focal position of the primary emission light focused by the light focusing member 23a, when the second irradiation module 70 (single fiber scope) is connected to the first light source module 20.

In the meantime, the relative distance L1, L2 is adjusted such that when either the bundle fiber scope or the single fiber scope is connected to the first light source module 20, the light beam diameter of the primary emission light, which is incident on the irradiation-side incidence portion 51, 71, is always greater than the core diameter of the single optical fiber 75a and is about 5 mm or less.

### [Optical Characteristic in First Radiation Module 50]

As described above, the first irradiation module 50 (bundle fiber scope) includes the bundle fiber 55a. Thus, the variation in light amount due to positional displacement of the primary emission light incident on the first irradiation module 50 is small. The positional displacement includes, for example, displacement of the optical axis on the first light source module 20 side relative to the optical axis on the first irradiation module 50 side, and displacement of the first irradiation module 50 relative to the first light source module 20 in the optical axis direction.

However, in the first irradiation module 50, the light intensity of the primary emission light, which is emitted from the light focusing member 23a, is nonuniform, and, in the case of a laser beam, the intensity distribution becomes a Gaussian distribution. In this case, a variance occurs among the amounts of light incident on the respective optical fibers of the bundle fiber 55a. The tendency of variance is propagated to the other end portion (emission portion) of the bundle fiber 55a. Consequently, a deviation occurs in the light intensity of the laser beam emitted from the bundle fiber 55a, and nonuniformity in luminance or nonuniformity in light distribution occurs in illumination light. In other words, nonuniformity occurs in the light distribution of illumination light. If nonuniformity occurs in the light distribution, there occurs a part where the light intensity of the primary emission light is high. In this part, there is concern that the adhesive which bundles the fibers is burnt by heat production. Therefore, it is necessary to uniformize the intensity distribution.

Thus, in the first irradiation module 50, the glass rod 51a, which uniformizes the intensity distribution, is mounted. In the glass rod 51a, the primary emission light, which is incident on the glass rod 51a, is reflected by the interface between the core portion and clad portion of the glass rod 51a. In addition, since the primary emission light is repeatedly reflected in the glass rod 51a, the intensity distribution of the primary emission light is uniformized. Thus, the primary emission light is made incident, with no variance, on the entirety of the incidence end face of the bundle fiber 55a. In addition, the intensity distribution of the primary emission light, which is incident on the incidence end face, is uniformized. Incidentally, the intensity distribution becomes more uniform as the number of times of reflection increases. In the meantime, for example, in the case of a lamp light source, it is general that convergent light with a large convergence angle is incident on the incidence end face. For the first irradiation module 50, convergent light, which can reflect light rays in the glass rod 51a, is suitable.

### [Optical Characteristic in Second Radiation Module 70]

As described above, in the second irradiation module 70 (single fiber scope), the diameter of the core portion is, for example, 50 *µ*m to 300 *µ*m. Thus, as regards the incidence end face at one end portion of the single optical fiber 75a and the primary emission light incident on this incidence end face, it is important to precisely set the position of incidence of the primary emission light on the incidence end face.

If external force acts on the light source-side connection hole portion 25, etc., there is concern that, for example, a small positional displacement occurs, such as displacement of the optical axis on the first light source module 20 side relative to the optical axis on the second irradiation module 70 side, or displacement of the second irradiation module 70 relative to the first light source module 20 in the optical axis direction. Consequently, there is concern that the incidence position is displaced, the mount of incident light varies, and, as a result, the luminance of illumination light becomes unstable. In order to suppress the displacement in the optical axis, it is desirable to diverge the primary emission light. In addition, in order to suppress the displacement in the optical axis direction, it is desirable that the primary emission light be a parallel beam. Besides, actually, the optical axis of the emission light emitted from the light focusing member 23a is misaligned from the ideal optical axis, due to restrictions, such as mounting precision, in the first light source module 20. In addition, as the relative distance L2 between the light focusing member 23a and the light focusing member 71a in the optical axis direction becomes longer, the amount of displacement of the incidence position of the primary emission light relative to the light focusing member 71a becomes larger, and the efficiency of connection becomes lower. Therefore, it is preferable that the relative distance L2 between the light focusing member 23a and the light focusing member 71a is as short as possible.

Thus, the collimation member 27 is mounted in the light source module 20, and the relative distance L2 is specified. By the collimation member 27, the primary emission light is diverged and converted to a parallel beam. By the relative distance L2, the incidence position is precisely set, the relative distance L2 between the light focusing member 23a and light focusing member 71a becomes shorter, the amount of displacement of the incidence position decreases, and a decrease in connection efficiency is prevented.

### [Summary of Optical Characteristic]

In general, the optimal primary emission light, which is incident on the second irradiation module 70, does not become the optimal primary emission light, which is incident on the first irradiation module 50, and it is not easy for the primary emission light to secure compatibility with both the first irradiation module 50 and the second irradiation module 70.

However, in the present embodiment, the light source unit 21 includes the LD which emits a laser beam having a smaller convergence angle than lamp light emitted from a lamp light source. By the length of the first irradiation-side incidence portion 51 and the length of the second irradiation-side incidence portion 71, the relative distances L1 and L2 are varied and adjusted in accordance with the first irradiation module 50 and second irradiation module 70. In addition, the position of the incidence end face of the glass rod 51a and the position of the light focusing member 71a, which are the incidence positions of the primary emission light, are adjusted. Thereby, the primary emission light easily secures compatibility with both the first irradiation module 50 and second irradiation module 70.

### [Operation]

### [Connection between First Light Source Module 20 and First Radiation Module 50 (Bundle Fiber Scope)]

As illustrated in FIG. 1B, when the first irradiation-side connection portion 53 is connected to the light source-side connection hole portion 25, the position of the incidence end face of the glass rod 51a relative to the light focusing member 23a is specified by the length of the first irradiation-side incidence portion 51 and by the mechanical connection between the first irradiation-side connection portion 53 and light source-side connection hole portion 25, such that the optical axis of the light focusing member 23a is made to agree with the optical axis of the glass rod 51a, the glass rod 51a is located in front of the focal point of the light focusing member 23a, and the relative distance L1 is specified.

The light-source light is emitted from the LD of the light source unit 21, and is converted to a parallel beam by the collimation member 27. Then, the parallel beam is focused by the light focusing member 23a on the glass rod 51a which is placed in front of the focal point of the light focusing member 23a, and the beam is incident on the glass rod 51a. The light intensity of the primary emission light, which is incident on the glass rod 51a, is nonuniform.

However, in the present embodiment, since the primary emission light is repeatedly reflected in the glass rod 51a, the intensity distribution of the primary emission light is uniformized, and the primary emission light is incident on the entirety of the incidence end face of the bundle fiber 55a with no variance. Thus, in the state in which the light intensity is uniformized, the primary emission light is incident on the bundle fiber 55a. In this state, the primary emission light is guided to the first irradiation-side emission unit 57 by the bundle fiber 55a. In addition, the primary emission light is emitted as illumination light by the optical conversion member 57a.

### [Connection between First Light Source Module 20 and Second Radiation Module 70 (Single Fiber Scope)]

As illustrated in FIG. 1C, when the second irradiation-side connection portion 73 is connected to the light source-side connection hole portion 25, the position of the light focusing member 71a relative to the light focusing member 23a is specified by the length of the second irradiation-side incidence portion 71 and by the mechanical connection between the second irradiation-side connection portion 73 and light source-side connection hole portion 25, such that the optical axis of the light focusing member 23a is made to agree with the optical axis of the light focusing member 71a, the light focusing member 71a is placed in rear of the focal point of the light focusing member 23a, and the relative distance L2 is specified.

The light-source light is emitted from the LD of the light source unit 21, and is converted to a parallel beam by the collimation member 27. Then, the parallel beam is focused by the light focusing member 23a on the light focusing member 71a which is located in rear of the focal point of the light focusing member 23a, and the beam is incident on the light focusing member 71a.

In the above, if external force acts on the light source-side connection hole portion 25, etc. , there is concern that, for example, a small positional displacement occurs, such as displacement of the optical axis on the first light source module 20 side relative to the optical axis on the second irradiation module 70 side, or displacement of the second irradiation module 70 relative to the first light source module 20 in the optical axis direction. Consequently, there is concern that the incidence position is displaced, the mount of incident light varies, and, as a result, the luminance of illumination light becomes unstable.

However, the collimation member 27 is disposed, and the relative distance L2 is specified. Thereby, the influence of displacement in the optical axis is suppressed, and the influence in displacement in the optical axis direction is suppressed.

The optical axis of the emission light emitted from the light focusing member 23a is misaligned from the ideal optical axis, due to restrictions, such as mounting precision, in the first light source module 20. However, since the relative distance L2 is short, it is possible to prevent an increase in displacement amount of the incidence position of the primary emission light relative to the light focusing member 71a, and a decrease in connection efficiency.

In this state, the primary emission light is incident on the single optical fiber. The primary emission light is guided to the second irradiation-side emission unit 77 by the single optical fiber. In addition, the primary emission light is emitted as illumination light by the optical conversion member 77a.

### [Advantageous Effects]

As described above, in the present embodiment, the light source-side connection hole portion 25 is made common to various kinds of irradiation modules, for example, the first irradiation module 50 and second irradiation module 70, which correspond to the first light source module 20 and have mutually different optical functions. In addition, in the present embodiment, the relative distance L1, L2 is adjusted as desired in accordance with the irradiation module 50, 70, which is connected to the first light source-side module 20. Therefore, in this embodiment, even if the respective irradiation modules 50 and 70 have mutually different optical functions, the irradiation modules 50 and 70 can exhibit performances.

When the first irradiation module 50 is the bundle fiber scope, the position of the incidence end face of the glass rod 51a relative to the light focusing member 23a is specified by the length of the first irradiation-side incidence portion 51 and by the mechanical connection between the first irradiation-side connection portion 53 and light source-side connection hole portion 25, such that the optical axis of the light focusing member 23a is made to agree with the optical axis of the glass rod 51a, the glass rod 51a is placed in front of the focal point of the light focusing member 23a, and the relative distance L1 is specified. Thereby, the primary emission light can be made incident on the glass rod 51a, the light intensity can be uniformized by the glass rod 51a, and the primary emission light can be made incident on the bundle fiber 55a in the state in which the light intensity is uniformized. Therefore, in the present embodiment, the occurrence of nonuniformity in light distribution of illumination light can be prevented, heat production can be prevented, and a target object can be irradiated with no variance.

When the second irradiation module 70 is the single fiber scope, the position of the light focusing member 71a relative to the light focusing member 23a is specified by the length of the second irradiation-side incidence portion 71 and by the mechanical connection between the second irradiation-side connection portion 73 and light source-side connection hole portion 25, such that the optical axis of the light focusing member 23a is made to agree with the optical axis of the light focusing member 71a, the light focusing member 71a is placed in rear of the focal point of the light focusing member 23a, and the relative distance L2 is specified. Thereby, even if external force acts on the light source-side connection hole portion 25, etc., it is possible to suppress the influence of displacement, such as displacement of the optical axis on the first light source module 20 side relative to the optical axis on the second irradiation module 70 side, or displacement of the second irradiation module 70 relative to the first light source module 20 in the optical axis direction. Therefore, the displacement of the incidence position can be prevented, the variation in amount of incident light can be prevented, and the luminance of the illumination light can be made stable.

In the present embodiment, the relative distance L1, L2 is adjusted when the first irradiation-side connection portion 53 is mechanically connected to the light source-side connection hole portion 25, and when the second irradiation-side connection portion 73 is mechanically connected to the light source-side connection hole portion 25. Thereby, in this embodiment, the relative distance L1, L2 can be adjusted without taking a lot of time and labor.

In particular, in this embodiment, the length of the irradiation-side incidence portion 51, 71 in the optical axis direction is different between the irradiation modules 50 and 70, and thereby the relative distance L1, L2 is adjusted. Thus, in this embodiment, the relative distance L1, L2 can be adjusted without taking a lot of time and labor.

In the present embodiment, the light focusing member 23a focuses the primary emission light on a desired part of the irradiation-side incidence portion 51, 71. Thereby, in this embodiment, in the case of either the first irradiation module 50 or the second irradiation module 70, the primary emission light can exactly be focused on the glass rod 51a or light focusing member 71a.

In the present embodiment, when the first irradiation module 50 (bundle fiber scope) is connected to the first light source module 20, the relative distance L1 is adjusted such that the glass rod 51a is located farther from the light focusing member 23a. Thereby, in this embodiment, the primary emission light can be made incident on the entirety of the incidence end face of the glass rod 51a.

In the present embodiment, when the second irradiation module 70 (single fiber scope) is connected to the first light source module 20, the relative distance L2 is adjusted such that the light focusing member 71a is located closer to the light focusing member 23a. Thereby, in this embodiment, the incidence position can be precisely set, the amount of displacement of the incidence position can be decreased, and a decrease in connection efficiency can be prevented.

In the present embodiment, the relative distance L1, L2 is adjusted such that when either the first irradiation module 50 (bundle fiber scope) or the second irradiation module 70 (single fiber scope) is connected to the first light source module 20, the light beam diameter of the primary emission light, which is incident on the irradiation-side incidence portion 51, 71, is always greater than the core diameter of the single optical fiber 75a and is about 5 mm or less. Thereby, such specific advantages can be obtained that the size of the first light source module 20 can be reduced and light can be made incident on the single optical fiber 75a.

In the present embodiment, 1.5 < focal distance f2 of the second lens/focal distance f1 of the first lens < 2.5. Thereby, the incidence NA toward the irradiation module side can be made smaller than the emission NA of the light-source light, and thus an allowance can be imparted to the tolerable incidence NA of the optical fiber, and a decrease in coupling efficiency due to a displacement in angle can be reduced.

In the meantime, in the present embodiment, the light focusing member 71a is disposed in rear of the focal point of the light focusing member 23a, but this embodiment does not need to be limited to this. The light focusing member 71a may be disposed in front of the focal point of the light focusing member 23a.

In the present embodiment, two kinds of irradiation modules (bundle fiber scope and single fiber scope) were used in the description. However, the kinds of the irradiation modules 50 and 70 are not limited to these.

In the present embodiment, one kind of first light source module 20 was used in the description. However, aside from the first light source module 20, a second light source module, which is optically different from the first light source module 20, may be disposed. The second light source module may emit, for example, LED light as light-source light.

For example, in a plurality of kinds of first irradiation modules 50 (bundle fiber scopes) with different diameters of bundle fibers 55a or different diameters of glass rods 51a, the relative distance L1 of each first irradiation module 50 may be adjusted, or the glass rod 51a may be disposed at such a position that the light intensity becomes uniform on average.

### [First Modification]

### [Structure]

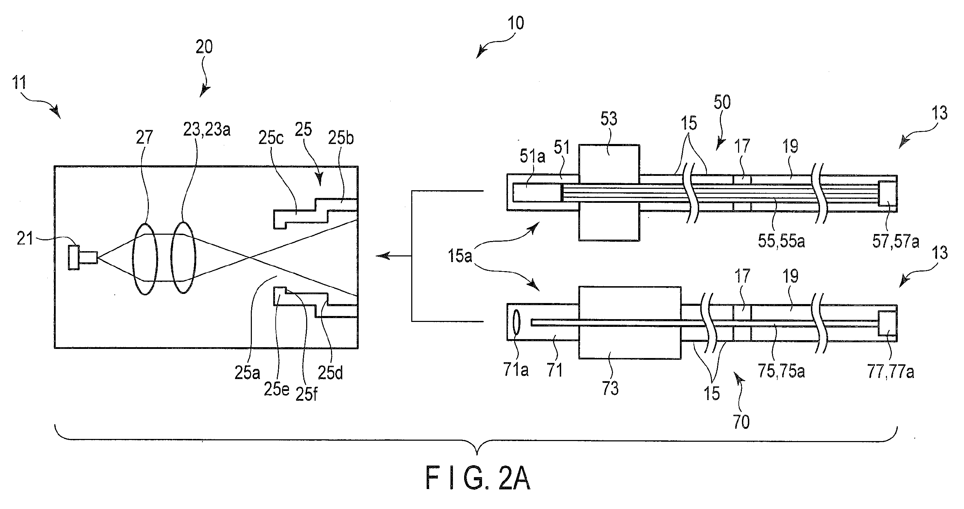

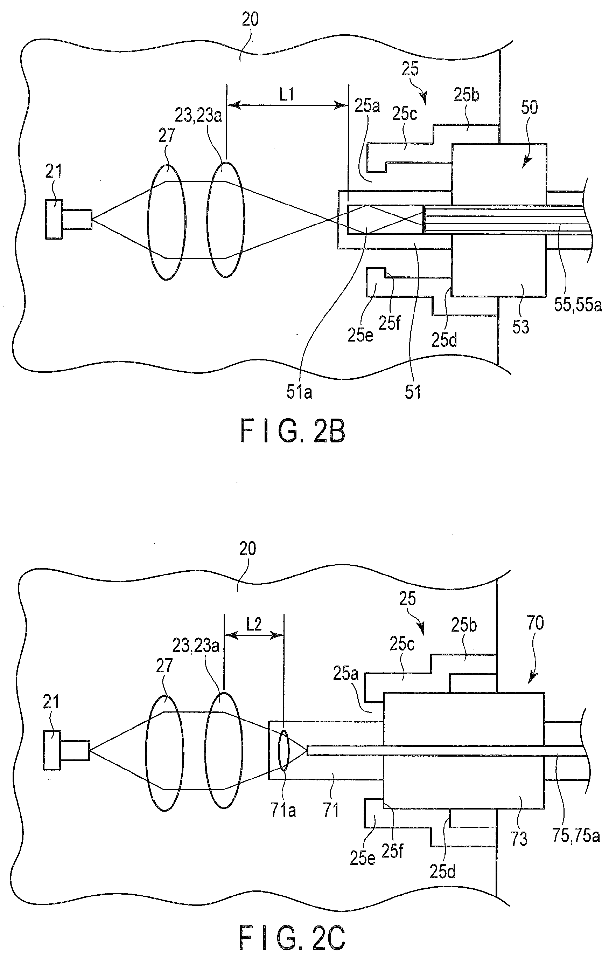

As illustrated in FIG. 2A, FIG. 2B and FIG. 2C, in the present modification, when the light source-side connection hole portion 25 is connected to the irradiation-side connection portion 53, 75, the position of connection of the irradiation-side connection portion 53, 75 to the light source-side connection hole portion 25 in the optical axis direction is different between the irradiation modules 50 and 70. Specifically, the position of connection of the first irradiation-side connection portion 53 to the light source-side connection hole portion 25 is different from the position of connection of the second irradiation-side connection portion 73 to the light source-side connection hole portion 25. Thereby, the relative distance L1, L2 is adjusted.

In this manner, as illustrated in FIG. 2A, FIG. 2B and FIG. 2C, in the present modification, the position of connection of the first irradiation-side connection portion 53 to the light source-side connection hole portion 25 and the position of connection of the second irradiation-side connection portion 73 to the light source-side connection hole portion 25 function as an adjustment mechanism which adjusts the relative distance L1, L2, as desired, in accordance with the irradiation module 50, 70, which is connected to the first light source module 20.

Thus, as illustrated in FIG. 2A, FIG. 2B and FIG. 2C, the light source-side connection hole portion 25 includes a first connection hole portion 25b to which the first irradiation-side connection portion 53 is detachably connected, and a second connection hole portion 25c to which the second irradiation-side connection portion 73 is detachably connected, and which has a less thickness than the first connection hole portion 25b. The first irradiation-side connection portion 53 is inserted/removed into/from the first connection hole portion 25b, and is detachably engaged with the first connection hole portion 25b. The second irradiation-side connection portion 73 is inserted/removed into/from the second connection hole portion 25c, and is detachably engaged with the second connection hole portion 25c. A center axis of the first connection hole portion 25b is disposed to agree with a center axis of the second connection hole portion 25c. The first connection hole portion 25b communicates with the second connection hole portion 25c in the center axis direction of the light source-side connection hole portion 25. The first connection hole portion 25b is disposed on the outside of the second connection hole portion 25c, and is disposed at a greater distance from the light focusing member 23a than the second connection hole portion 25c.

As illustrated in FIG. 2A, FIG. 2B and FIG. 2C, the first irradiation-side incidence portion 51 has the same length as the second irradiation-side incidence portion 71. Each of the first irradiation-side incidence portion 51 and second irradiation-side incidence portion 71 is thinner than the second connection hole portion 25c.

For example, each of the first irradiation-side connection portion 53 and second irradiation-side connection portion 73 has a cylindrical shape, and each of the first connection hole portion 25b and second connection hole portion 25c has a cylindrical shape. The first irradiation-side connection portion 53 is thicker than second irradiation-side connection portion 73.

As illustrated in FIG. 2A, FIG. 2B and FIG. 2C, since the first connection hole portion 25b is thicker than second connection hole portion 25c, a first end face 25d having a planar and annular shape is formed at a boundary portion between the first connection hole portion 25b and second connection hole portion 25c. The first end face 25d is disposed in a direction perpendicular to the center axis of the light source-side connection hole portion 25. When the first irradiation-side connection portion 53 is connected to the first connection hole portion 25b, a distal end face of the first irradiation-side connection portion 53 abuts on the first end face 25d, and thus the first end face 25d functions as a stopper surface which prevents the first irradiation-side connection portion 53 from being passed through the first connection hole portion 25b and inserted into the second connection hole portion 25c. When the first irradiation-side connection portion 53 is connected to the first connection hole portion 25b, the distal end face of the first irradiation-side connection portion 53 abuts on the first end face 25d, and thus the first end face 25d, and thus the first end face 25d positions the first irradiation-side incidence portion 51 such that the optical axis of the light focusing member 23a agrees with the optical axis of the glass rod 51a, the glass rod 51a is placed in front of the focal point of the light focusing member 23a, and the relative distance L1 is specified.

As illustrated in FIG. 2A, FIG. 2B and FIG. 2C, a distal end portion 25e of the second connection hole portion 25c is bent toward the center axis so as to function as an inner flange portion. An inner end face 25f of the distal end portion 25e is formed to have a planar and annular shape, and is disposed in a direction perpendicular to the center axis of the light source-side connection hole portion 25. When the second irradiation-side connection portion 73 is connected to the second connection hole portion 25c, a distal end face of the second irradiation-side connection portion 73 abuts on the inner end face 25f, and thus the inner end face 25f functions as a stopper surface which prevents the second irradiation-side connection portion 73 from being passed through the second connection hole portion 25c. When the second irradiation-side connection portion 73 is connected to the second connection hole portion 25c, the distal end face of the second irradiation-side connection portion 73 abuts on the inner end face 25f, and thus the inner end face 25f positions the second irradiation-side incidence portion 71 such that the optical axis of the light focusing member 23a agrees with the optical axis of the light focusing member 71a, the light focusing member 71a is placed in rear of the focal point of the light focusing member 23a, and the relative distance L2 is specified. The distal end portion 25e includes a through-hole portion 25a.

### [Advantageous Effects]

In the present modification, also in the second irradiation module 70 (single fiber scope), the distance from the light source-side connection hole portion 25 to the light focusing member 71a can be shortened. In this modification, when external force acts on the light source-side connection hole portion 25, etc., it is possible to prevent the optical axis of the light focusing member 71a from being displaced, with the light source-side connection hole portion 25 functioning as a fulcrum, and the optical coupling efficiency can further be improved.

### [Second Modification]

### [Structure]

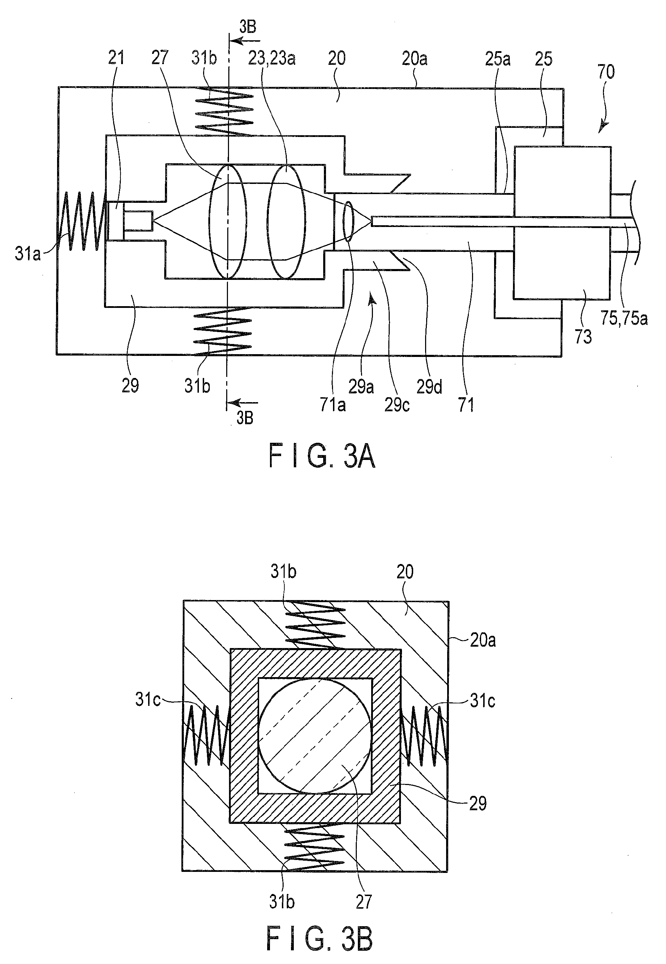

In FIG. 3A, although the second irradiation module 70 is used by way of example, the same applies to the first irradiation module 50.

As illustrated in FIG. 3A and FIG. 3B, the first light source module 20 further includes a holding member 29 which integrally holds the light source unit 21, collimation member 27 and light source-side emission unit 23 such that the light source unit 21, collimation member 27 and light source-side emission unit 23 are fixed. The holding member 29 functions as a lens frame, and holds the light source unit 21, collimation member 27 and light source-side emission unit 23 within the holding member 29.

As illustrated in FIG. 3A and FIG. 3B, the holding member 29 includes a guide portion 29a which guides the irradiation-side incidence portion 51, 71 when the irradiation-side connection portion 53, 73 is connected to the light source-side connection hole portion 25, such that the irradiation-side incidence portion 51, 71 is disposed coaxial with the light source-side emission unit 23. The guide portion 29a includes a cylinder portion 29c into/from which the irradiation-side incidence portion 51, 71 is inserted/removed, and with which the irradiation-side incidence portion 51, 71 is engaged. The cylinder portion 29c communicates with the inside of the holding member 29 in the insertion direction. The center axis of the cylinder portion 29c is disposed to agree with the center axis of the light focusing member 23a. The inside diameter and inner shape of the cylinder portion 29c are substantially identical to the outside diameter and outer shape of the irradiation-side incidence portion 51, 71. The cylinder portion 29c includes an insertion/removal opening portion 29d which is provided at one end portion of the cylinder 29c and through which the irradiation-side incidence portion 51, 71 is inserted/removed into/from the cylinder portion 29c. The insertion/removal opening portion 29d becomes gradually narrower in the insertion direction toward the inside of the holding member 29. The insertion/removal opening portion 29d is wider than, for example, the outside diameter of the second irradiation-side incidence portion 71, in consideration of an optical axis displacement in the incidence end face, which occurs due to the mechanical connection between, for example, the light source-side connection hole portion 25 and second irradiation-side connection portion 73. The minimum diameter of the insertion/removal opening portion 29d is close to, for example, the outside diameter of the second irradiation-side incidence portion 71 within such a range that insertion is not hindered. This point is similarly applicable to the first irradiation-side incidence portion 51.

As illustrated in FIG. 3A and FIG. 3B, the first light source module 20 further includes a first urging member 31a which urges the holding member 29 in the insertion/removal direction and positions the holding member 29 in the insertion/removal direction; a second urging member 31b which urges the holding member 29 in a first perpendicular direction which is perpendicular to the insertion/removal direction, and positions the holding member 29 in the first perpendicular direction; and a third urging member 31c which urges the holding member 29 in a second perpendicular direction which is perpendicular to the insertion/removable direction and the first perpendicular direction, and positions the holding member 29 in the second perpendicular direction. The first urging member 31a, second urging member 31b and third urging member 31c include, for example, coil springs. One end portion of the first urging member 31a is fixed to an inner peripheral surface of an armor body 20a of the first light source module 20, and the other end portion of the first urging member 31a is fixed to an outer peripheral surface of the holding member 29. This point is similarly applicable to the second urging member 31b and third urging member 31c. The first urging member 31a is disposed coaxial with the center axis of the light focusing member 23a. The second urging members 31b are disposed on both sides of the holding member 29 in the first perpendicular direction. The second urging members 31b are disposed on the same axis. The third urging members 31c are disposed on both sides of the holding member 29 in the second perpendicular direction. The third urging members 31c are disposed on the same axis.

### [Advantageous Effects]

In the present modification, by the guide portion 29a, the irradiation-side incidence portion 51, 71 can easily be disposed coaxial with the light source-side emission unit 23. In this modification, by the cylinder portion 29c, the irradiation-side incidence portion 51, 71 can be protected from the outside.

In the present modification, by the first, second and third urging members 31a, 31b and 31c, the position of the holding member 29 including the light source unit 21, collimation member 27 and light source-side emission unit 23 can be adjusted relative to the irradiation-side incidence portion 51, 71. In addition, by the first, second and third urging members 31a, 31b and 31c, the holding member 29 is movable in the three directions when the irradiation-side incidence portion 51, 71 is inserted into the cylinder portion 29c, and it is possible to prevent the holding member 29, irradiation-side incidence portion 51, 71 and cylinder portion 29c from damaging each other due to the insertion. Furthermore, the optical coupling efficiency can be enhanced in the three direction.

In the meantime, in the present modification, since the irradiation-side incidence portion 51, 71 is inserted into the guide portion 29a regardless of the optical function of the irradiation module 50, 70, it is preferable that the outer shapes and outside diameters of the irradiation-side incidence portions 51 and 71 are substantially identical. It is preferable that the length of the guide portion 29a is adjusted in accordance with the irradiation module having the longest relative distance L1, L2.

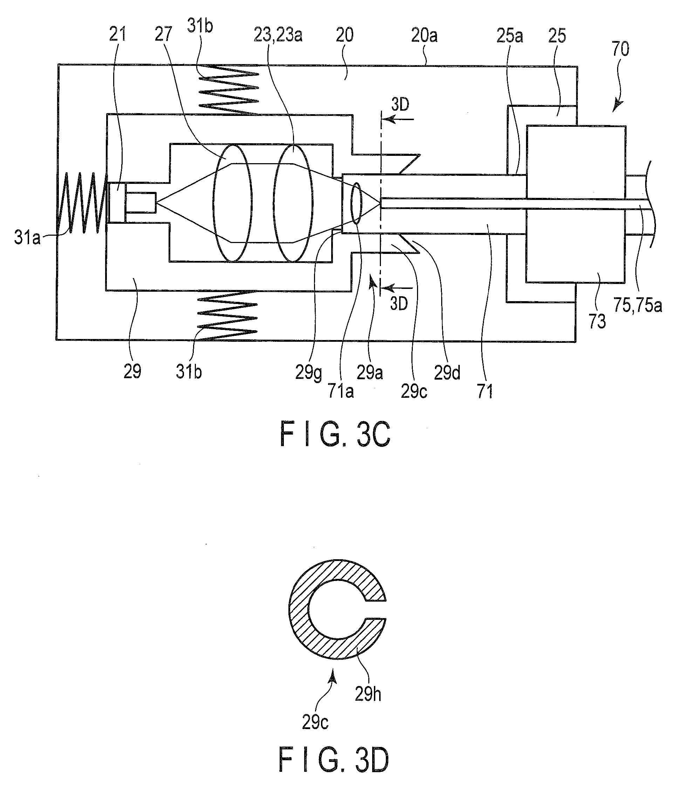

As illustrated in FIG. 3C, the cylinder portion 29c may include a cylinder-side abutment surface 29g which is provided on the other end portion of the cylinder portion 29c. Assuming that the relative distance L2 is shortest, when the second irradiation-side connection portion 73 is inserted into the cylinder portion 29c, the distal end face of the second irradiation-side connection portion 73 abuts on the cylinder-side abutment surface 29g, and thereby the cylinder-side abutment surface 29g functions as a stopper surface which prevents the second irradiation-side incidence portion 71 from being inserted into the holding member 29. When the distal end face of the second irradiation-side connection portion 73 abuts on the cylinder-side abutment surface 29g, the relative distance L1 is specified.

Thereby, in the present modification, the shortest relative distance (L2 in this example) can exactly be specified.

As illustrated in FIG. 3C and FIG. 3D, the cylinder portion 29c may include a split sleeve 29h which applies stress to the irradiation-side incidence portion 51, 71 from the outer peripheral side of the cylinder portion 29c toward the central side of the cylinder portion 29c, and positions and fixes the irradiation-side incidence portion 51, 71. For the purpose of the stress, the inside diameter of the split sleeve 29h is slightly less than the outside diameter of the irradiation-side incidence portion 51, 71. The split sleeve 29h has a C-shaped cross section in a direction perpendicular to the center axis of the split sleeve 29h.

In the present modification, by the split sleeve 29h, the influence of looseness due to the engagement between the irradiation-side incidence portion 51, 71 and the split sleeve 29h can be suppressed, the irradiation-side incidence portion 51, 71 can be positioned and fixed, the positional displacement of the irradiation-side incidence portion 51, 71 relative to the light focusing member 23a can be prevented, and the optical coupling efficiency can be enhanced.

### [Second Embodiment]

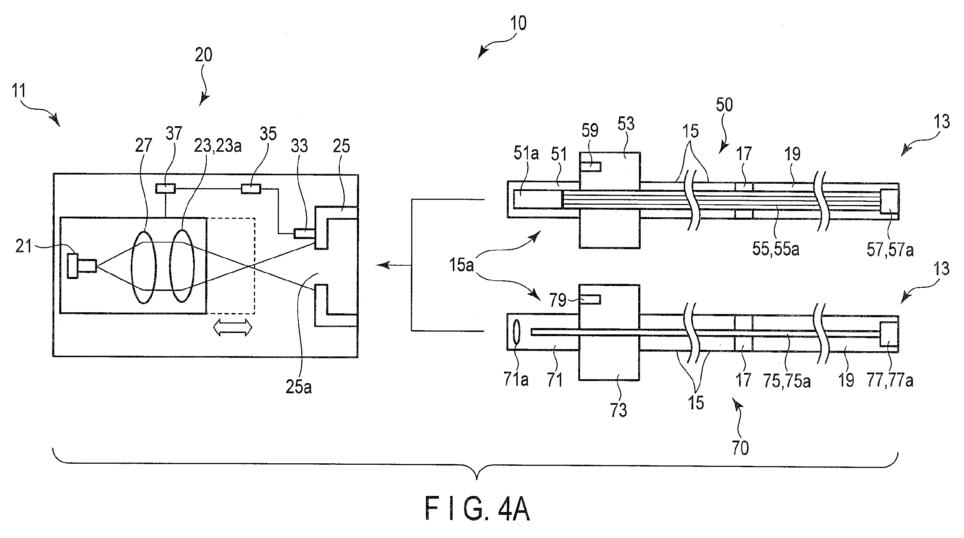

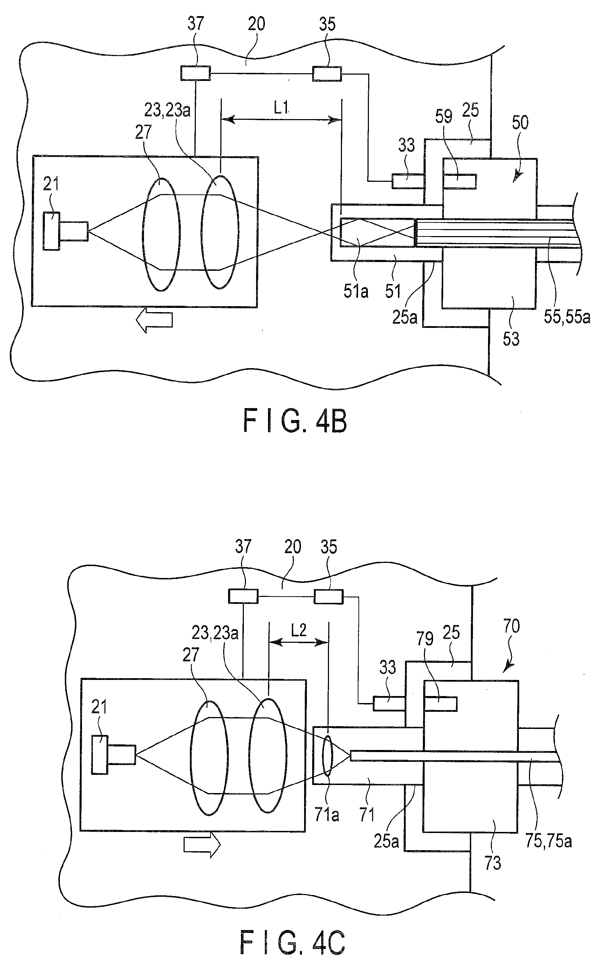

With reference to FIG. 4A, FIG. 4B and FIG. 4C, only different points from the first embodiment will be described.

In the present embodiment, the position of connection of the first irradiation-side connection portion 53 to the light source-side connection hole portion 25 is identical to the position of connection of the second irradiation-side connection portion 73 to the light source-side connection hole portion 25. The second irradiation-side connection portion 73 has the same length, same thickness and outer shape as the first irradiation-side connection portion 53.

In this embodiment, the first irradiation-side incidence portion 51 has the same length, same thickness and same outer shape as the second irradiation-side incidence portion 71.

The first irradiation module 50 includes a first storage unit 59 which stores information to the effect that the irradiation module is the first irradiation module 50. When the first irradiation-side connection portion 53 is connected to the light source-side connection hole portion 25, the first storage unit 59 transmits the information to a determination unit 33 which is disposed in the first light source module 20.

The second irradiation module 70 includes a second storage unit 79 which stores information to the effect that the irradiation module is the second irradiation module 70. When the second irradiation-side connection portion 73 is connected to the light source-side connection hole portion 25, the second storage unit 79 transmits the information to the determination unit 33 which is disposed in the first light source module 20.

In this manner, each irradiation module 50, 70 includes the storage unit 59, 79 which stores the information to the effect that the irradiation module is the irradiation module 50, 70. When each irradiation module 50, 70 is connected to the first light source module 20, each irradiation module 50, 70 transmits the information from the storage unit 59, 79 to the first light source module 20, so that the first light source module 20 can determine the kind (optical function) of the irradiation module 50, 70 connected to the first light source module 20.

The first light source module 20 further includes the determination unit 33 which determines the irradiation module 50, 70 connected to the first light source module 20. Based on the information stored in the storage unit 59, 79, the determination unit 33 determines whether the irradiation module connected to the first light source module 20 is the first irradiation module 50 or the second irradiation module 70.

The first light source module 20 further includes a control unit 35 which controls a moving unit 37 (to be described later), based on a determination result of the determination unit 33. The control unit 35 may control the light source unit 21 such that the light source unit 21 is driven based on the determination result of the determination unit 33.

In the present embodiment, the first light source module 20 further includes a moving unit 37 which moves the light source-side emission unit 23 in the optical axis direction in accordance with the irradiation module 50, 70 connected to the first light source module 20, such that the relative distance L1, L2 is adjusted in accordance with the irradiation module 50, 70 connected to the first light source module 20 when the light source-side connection hole portion 25 is connected to the irradiation-side connection portion 53 , 73. The moving unit 37 is controlled by the control unit 35, and moves the light source-side emission unit 23 in accordance with each irradiation module 50, 70, based on the above-described determination result. The moving unit 37 may move not only the light source-side emission unit 23, but also the light source-side emission unit 23, collimation member 27 and light source unit 21 as a single unit. This moving unit 37 includes, for example, a stepping motor. In this manner, the moving unit 37 functions as an adjusting mechanism which adjusts the relative distance L1, L2, as desired, in accordance with the irradiation module 50, 70 connected to the first light source module 20.

### [Advantageous Effects]

In the present embodiment, in the first irradiation module 50 and second irradiation module 70, the irradiation-side incidence portion 51, 71 can be made common, and the irradiation-side connection portion 53, 73 can be made common. Thereby, in this embodiment, commonalty and compatibility can be provided to all irradiation modules 50, 70, with respect to the light source-side connection hole portion 25, a case for storing the irradiation module 50, 70, and a cleaner for cleaning the irradiation module 50, 70.

In the present embodiment, the relative distances L1 and L2, the position of the incidence end face of the glass rod 51a relative to the light focusing member 23a, and the position of the light focusing member 71a relative to the light focusing member 23a, are different in accordance with the kind (optical function) of the irradiation module 50, 70. In this case, too, in the present embodiment, these distances and positions can be finely adjusted by the moving unit 37, and even if the respective irradiation modules 50 and 70 have mutually different optical functions, the respective irradiation modules 50 and 70 can sufficiently and easily exhibit the performances.

In the present embodiment, by the storage units 59 and 79, determination unit 33 and control unit 35, the relative distance L1, L2 can be adjusted in accordance with the irradiation module 50, 70 when the irradiation module 50, 70 is connected to the first light source module 20.

In the meantime, although the moving unit 37 is controlled and moved by the control unit 35, the moving unit 37 does not need to be restricted to this, and the moving unit 37 may be moved manually.

The present invention is not limited directly to the above-described embodiment. At the stage of practicing the invention, the structural elements may be modified and embodied without departing from the spirit of the invention. Further, various inventions may be made by suitably combining a plurality of structural elements disclosed in the embodiments.

## Claims

1. An endoscope light source system in which a light source module and an irradiation module, which is mechanically detachably attached to the light source module, are combined and thereby illumination light corresponding to a purpose of use is emitted,
the light source module comprising:
a light source unit configured to emit light-source light;
a light source-side emission unit configured to convert an optical characteristic of the light-source light, and to emit the light with the converted optical characteristic as primary emission light; and
a light source-side connection hole portion disposed on an optical axis of the light source-side emission unit, and made common to various kinds of the irradiation modules which have mutually different optical functions, and
the irradiation module comprising:
an irradiation-side incidence portion on which the primary emission light emitted from the light source-side emission unit is made incident;
an irradiation-side connection portion configured to be connected to the light source-side connection hole portion, such that the irradiation-side incidence portion is disposed coaxial with the light source-side emission unit, and the primary emission light emitted from the light source-side emission unit is made incident on the irradiation-side incidence portion;
a light guide member configured to guide the primary emission light made incident on the irradiation-side incidence portion; and
an irradiation-side emission unit configured to convert an optical characteristic of the primary emission light guided by the light guide member, and to emit the light with the converted optical characteristic as the illumination light to an outside,
wherein a relative distance between the light source-side emission unit and the irradiation-side incidence portion in an optical axis direction is adjusted, as desired, in accordance with the irradiation module which is connected to the light source module.

2. The endoscope light source system according to Claim 1, wherein a size of an incidence end face of the light guide member, on which the primary emission light is made incident, is different between the irradiation modules.

3. The endoscope light source system according to Claim 2, wherein the light source module is connectable to either the irradiation module in which the light guide member includes a bundle fiber which is formed by bundling a plurality of optical fiber strands, or to the irradiation module in which the light guide member includes a single optical fiber.

4. The endoscope light source system according to Claim 1, wherein the relative distance is adjusted when the irradiation-side connection portion is mechanically connected to the light source-side connection hole portion.

5. The endoscope light source system according to Claim 4, wherein a length of the irradiation-side incidence portion relative to the irradiation-side connection portion in the optical axis direction is different between the irradiation modules, and thereby the relative distance is adjusted.

6. The endoscope light source system according to Claim 4, wherein when the light source-side connection hole portion is connected to the irradiation-side connection portion, a position of connection of the irradiation-side connection portion to the light source-side connection hole portion in the optical axis direction is different between the irradiation modules, and thereby the relative distance is adjusted.

7. The endoscope light source system according to Claim 4, wherein the light source module includes a moving unit configured to move the light source-side emission unit in the optical axis direction in accordance with the irradiation module connected to the light source module, such that the relative distance is adjusted in accordance with the irradiation module connected to the light source module when the light source-side connection hole portion is connected to the irradiation-side connection portion.

8. The endoscope light source system according to Claim 4, wherein the light source-side emission unit includes a light focusing member configured to focus the primary emission light on a desired part of the irradiation-side incidence portion when the light source-side connection hole portion is connected to the irradiation-side connection portion.

9. The endoscope light source system according to Claim 8, wherein the relative distance is adjusted such that the irradiation-side incidence portion is located farther from a focal position of the primary emission light focused by the light focusing member, when the irradiation module including a bundle fiber is connected to the light source module, and
the relative distance is adjusted such that the irradiation-side incidence portion is located closer to the focal position of the primary emission light focused by the light focusing member, when the irradiation module including a single optical fiber is connected to the light source module.

10. The endoscope light source system according to Claim 8, wherein the relative distance is adjusted such that when either the irradiation module including a bundle fiber or the irradiation module including a single optical fiber is connected to the light source module, a light beam diameter of the primary emission light, which is incident on the irradiation-side incidence portion, is always greater than a core diameter of the single optical fiber and is about 5 mm or less.

11. The endoscope light source system according to Claim 8, wherein the light source module further includes a collimation member configured to convert the light-source light to a parallel beam,
the collimation member includes a first lens, and the light focusing member includes a second lens, and
1.5 < focal distance f2 of the second lens/focal distance f1 of the first lens < 2.5.

12. The endoscope light source system according to Claim 8, wherein the light source module further includes a holding member configured to integrally hold the light source unit and the light source-side emission unit such that the light source unit and the light source-side emission unit are fixed,
the holding member includes a guide portion configured to guide the irradiation-side incidence portion when the irradiation-side connection portion is connected to the light source-side connection hole portion, such that the irradiation-side incidence portion is disposed coaxial with the light source-side emission unit, and
the guide portion includes a cylinder portion into which the irradiation-side incidence portion is inserted.

13. The endoscope light source system according to Claim 12, wherein the cylinder portion includes a split sleeve configured to apply stress to the irradiation-side incidence portion from an outer peripheral side of the cylinder portion toward a central side of the cylinder portion, and to position and fix the irradiation-side incidence portion.

14. The endoscope light source system according to Claim 12, wherein the light source module further includes a first urging member configured to urge the holding member in an insertion/removal direction and to position the holding member in the insertion/removal direction; a second urging member configured to urge the holding member in a first perpendicular direction which is perpendicular to the insertion/removal direction, and to position the holding member in the first perpendicular direction; and a third urging member configured to urge the holding member in a second perpendicular direction which is perpendicular to the insertion/removable direction and the first perpendicular direction, and to position the holding member in the second perpendicular direction.
